# EUROPEAN PATENT APPLICATION

(11) **EP 2 044 974 A1**
(43) Date of publication of application: **08.04.2009**
(21) Application number: 07460024.8
(22) Date of filing: 03.10.2007
(51) Int. Cl.: A61N 5/06

(54) **Light-emitting diode (LED) light therapy device**

(71) Applicant: Aracaria B.V., 1017 JP Amsterdam (NL)
(72) Inventor: Vibor Mulic, 00-730 Warszawa (PL)
(74) Representative: Viering, Jentschura & Partner

(57) **Abstract**

The light-emitting diode (LED) light therapy device is used especially in the preventive treatment, rehabilitation and the treatment of many diseases.

The light-emitting diode (LED) light therapy device is equipped with a matrix board (1) and a control system board (2), connected to a keypad (3), while the matrix board (1) comprises light-emitting diodes (LEDs) in the red light wavelength range (LED-R) (4) and the infrared light wavelength range (LED-IR) (5), and the control system board (2) is equipped with a pre-programmed processor (11), which controls the LED diodes within the red light wavelength range (LED-R) (4) and the infrared wavelength range (LED-IR) (5), a buzzer (9) and indicator lamps (10).

## Description

The subject of the invention is a light-emitting diode (LED) light therapy device to be used especially in the preventive treatment, rehabilitation and the treatment of many diseases.

The therapeutic effect of LED light exposure is produced by the LED radiation modulation of suitable frequencies. These frequencies produce resonance with organism cell own frequencies, stimulate those cells and produce temporary depolarisation thereof. For different kinds of therapy, diversified values of the light modulating frequency ranging from 0 to 8000 Hz are used. The optimum pulsation frequency is dependent on the type of exposed tissues and the nature of disorders.

The light therapy efficacy is dependent on an organism's reaction to a radiation dose. The efficacy is determined by the Arndt-Schultz-Oshiro law, which says that a radiation dose between 0.1 and 16 J/cm² produces a positive reaction of an organism, while the maximum reaction occurs between 4 and 12 J/cm². Doses of 4 J/cm² are used most frequently, which corresponds to power density of 50 mW/cm². The light wavelength allowing light penetration into a human body is between 550 and 950 nm, while its optimum is about 700 nm.

There is a light therapy device known from the Polish P-372056 patent description, which consists of a control system and light points that are characterized by colour light points, favourably multi-colour diodes, favourably RGB LED diodes around the light therapy location. The light therapy device has lighting points arranged at least in one row along the longitudinal axis sides of a bath, favourably a hydro-massage one. The light therapy device has lighting points arranged symmetrically in the shower cabin walls and ceiling.

The LED light therapy device, according to the invention, is characterized by its equipment comprising a matrix board and control system board connected to a keypad. The matrix board comprises light-emitting diodes (LEDs) in the red light wavelength range (LED-R) and the infrared light wavelength range (LED-IR). It is equipped with a power supply socket, a battery socket and cables connecting the matrix board with the control system board. The control system board is equipped with a pre-programmed processor, which controls the LED diodes within the red light wavelength range (LED-R) and the infrared wavelength range (LED-IR), a buzzer (9) and indicator lamps. As LED diodes, diodes the structure of which is based on GaAlAs semiconductors generating electromagnetic waves within the red light wavelength range and GaAlAs/GaAs semiconductors generating electromagnetic waves within the close infrared light wavelength range are used in the device, and diodes of the wavelength favourably of 625-680 nm and radiation angle of 10-30 degrees are used as the red light wavelength LEDs (LED-R), while diodes of the wavelength favourably of 880 nm and radiation angle of 30-45 degrees are used as the infrared light wavelength LEDs (LED-IR). The matrix board favourably comprises 108 LED diodes, whereas the quantitative ratio of the red light wavelength LEDs (LED-R) to the infrared light wavelength LEDs (LED-IR) is 1:1. The red light wavelength LED diodes (LED-R) and the infrared light wavelength LED diodes (LED-IR) are alternately arranged on the matrix board in a chequered pattern. The LED diodes within the red light wavelength range (LED-R) and the infrared wavelength range (LED-IR), the buzzer and the indicator lamps are controlled via the keypad equipped with the ON/OFF button used to switch on/off the device, the TIME button used to select the exposure time, the MODE button used to select the exposure program, the START button used to start the exposure process with the parameters already selected by the TIME and MODE buttons, and points which light from the indicator lights goes through.

The buzzer generates an aural signal that indicates any changes of the time settings selected by the TIME button, the device operating frequency adjusted by the MODE button, the beginning of exposure by the START button and the completion of exposure. The buzzer also indicates a low battery.

The indicator lamps alternately indicate the exposure time selected by the TIME button, and then the exposure program number selected by the MODE button dependent on the desired length of the waves emitted by the red light wavelength LED diodes (LED-R) and the infrared light wavelength LED diodes (LED-IR), the type of radiation (continuous or pulsating) and frequency.

The solution under the invention allows a user to make local exposure of the affected areas of the body, finally resulting in a positive therapeutic and preventive reaction in the whole human organism by accelerating its healing, reconstructing the disturbed functions of the organism and increasing its immunity. The LED diodes applied in the device under the invention emit light of the most physiological, therapeutic effect. The combination of the red light wavelengths and infrared light wavelengths with the pulsatory emission thereof creates the best conditions for the treatment, preventive treatment and rehabilitation.

The subject of the invention is shown as an example of a ready-made device in the following figures:

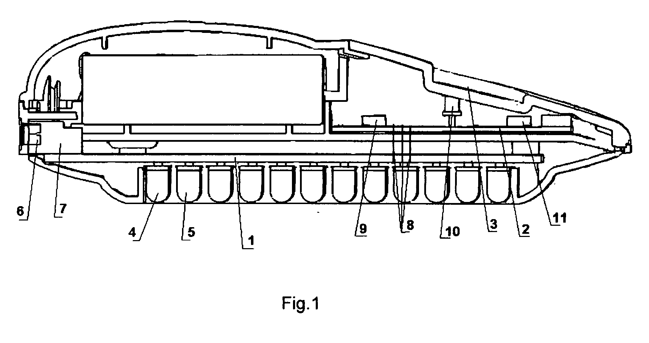

Fig. 1 shows a longitudinal section of the device,

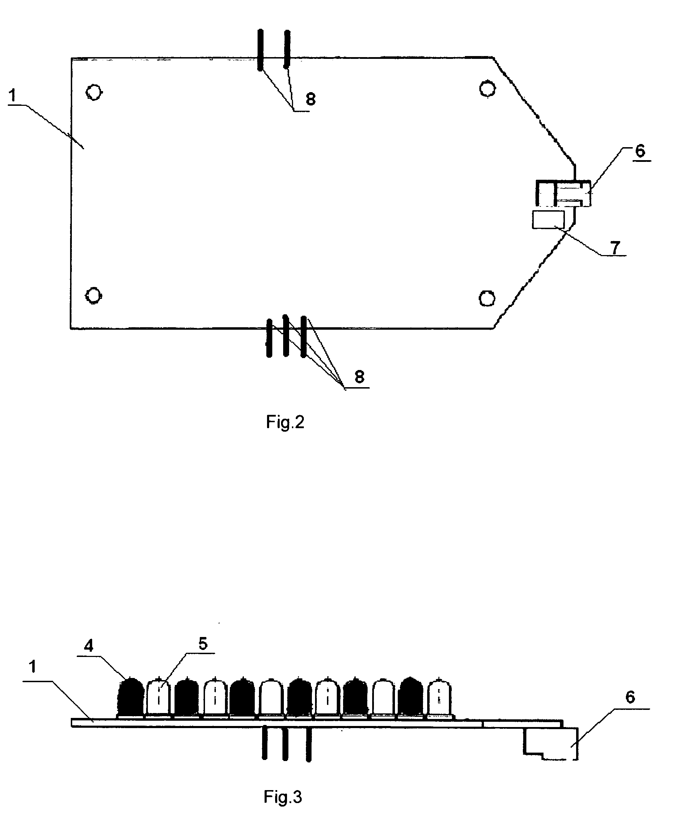

Fig. 2 shows the matrix board of the device in a top view,
Fig. 3 shows the matrix board of the device in a longitudinal section,

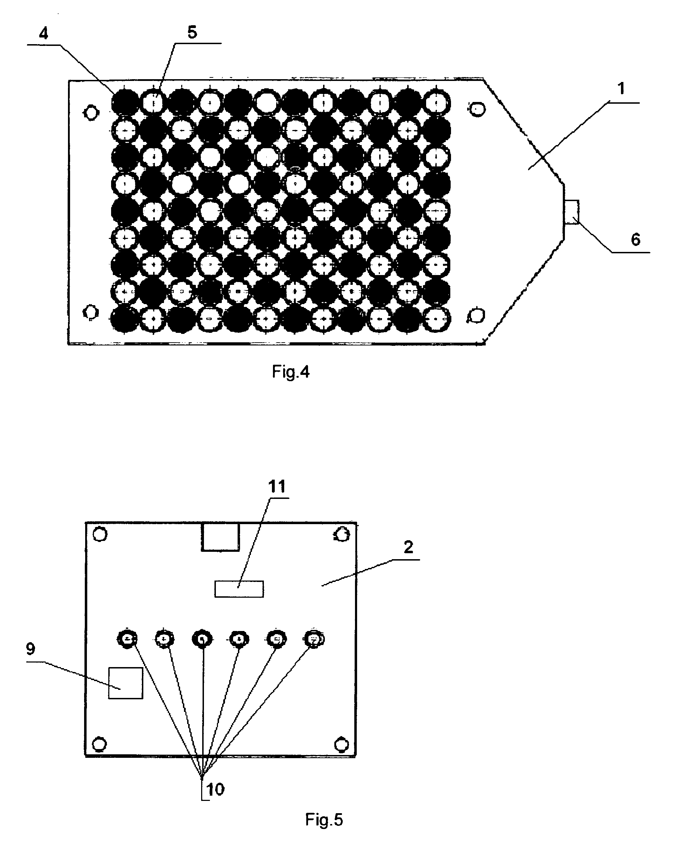

Fig. 4 shows the matrix board of the device in a bottom view,
Fig. 5 shows the control system board of the device in a top view,

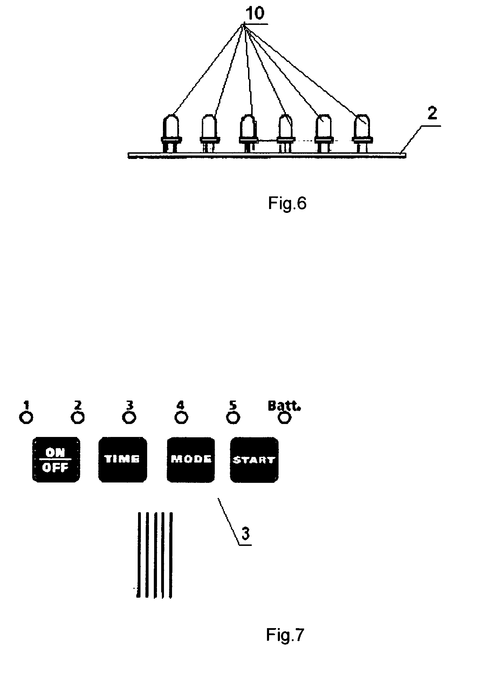

Fig. 6 shows the control system board of the device in a longitudinal section,
Fig. 7 shows a view of the keyboard of the device.

The LED light therapy device is equipped with a matrix board 1 and a control system board 2 connected to a keypad 3. The matrix board 1 comprises light-emitting diodes (LEDs) in the red light wavelength range (LED-R) 4 and the infrared light wavelength range (LED-IR) 5. It is equipped with a power supply socket 6, a battery socket 7 and cables 8 connecting the matrix board 1 with the control system board 2. The control system board (2) is equipped with a pre-programmed processor (11), which controls the LED diodes within the red light wavelength range (LED-R) (4) and the infrared wavelength range (LED-IR) (5), a buzzer (9) and indicator lamps (10). The matrix board 1 comprises 108 LED diodes, including 54 red light wavelength range LEDs (LED-R) 4 and 54 infrared light wavelength range LEDs (LED-IR) 5. The red light wavelength LED diodes (LED-R) 4 and the infrared light wavelength LED diodes (LED-IR) 5 are alternately arranged on the matrix board 1 in a chequered pattern. The LED diodes within the red light wavelength range (LED-R) (4) and the infrared wavelength range (LED-IR) (5), the buzzer (9) and the indicator lamps (10) are controlled via the keypad (3) equipped with the ON/OFF button used to switch on/off the device, the TIME button used to select the exposure time, the MODE button used to select the exposure program, the START button used to start the exposure process with the parameters already selected by the TIME and MODE buttons, and points through which light reaches the indicator lights (10) marked as numbers 1-5 on the keypad (3).

After switching the device under the invention with the ON/OFF button on the keypad (3), one of the five exposure times is selected by the TIME button, which turns on an indicator lamp or lamps (10) marked as numbers 1-5 on the keypad (3) depending on the exposure time, for example:
for the exposure time of 2 min. - the indicator lamp (10) marked as number 1 illuminates on the keypad (3),
for the exposure time of 4 min. - the indicator lamps marked as numbers 1, 2 illuminate on the keypad (3),
for the exposure time of 6 min. - the indicator lamps (10) marked as numbers 1, 2, 3 illuminate on the keypad (3),
for the exposure time of 8 min. - the indicator lamps (10) marked as numbers 1, 2, 3, 4 illuminate on the keypad (3),
for the exposure time of 10 min. - the indicator lamps (10) marked as numbers 1, 2, 3, 4, 5 illuminate on the keypad (3).

After setting the exposure time, the MODE button on the keypad (3) is used to select one of the five exposure programs of the following parameters, for example:
Program 1.
   Continuous radiation - the indicator lamp (10) marked as number 1 illuminates on the keypad (3)
Program 2.
   10 Hz frequency pulsating radiation - the indicator lamp (10) marked as number 2 illuminates on the keypad (3)
Program 3.
   600 Hz frequency pulsating radiation - the indicator lamp (10) marked as number 3 illuminates
Program 4.
   3 kHz frequency pulsating radiation - the indicator lamp (10) marked as number 4 illuminates,
Program 5.
   8 kHz frequency pulsating radiation - the indicator lamp (10) marked as number 5 illuminates.

The exposure by the device under the invention starts when the START button on the keypad (3) is pressed. During the exposure, the TIME, MODE and START buttons on the keypad (3) are disabled. The exposure completes automatically after a selected exposure time ends and it is indicated by an aural signal. Pressing any of the TIME, MODE and START buttons on the keypad (3) is also indicated by an aural signal. After the completed exposure, the ON/OFF button on the keypad (3) should be pressed.

### Example

In order to select the exposure time of 4 minutes and the exposure program of the parameters corresponding to radiation during which 600 Hz pulsating light is emitted, the device is switched on by the ON/OFF button on the keypad (3). After switching the device on, the indicator light (10) marked as number 1 illuminates, which indicates that the exposure time of 2 minutes is automatically set. Then, the TIME button is pressed, which sets the exposure time longer by 2 minutes, i.e. the required time of exposure of 4 minutes, which is indicated by two indicator lamps (10) marked as numbers 1 and 2 illuminating for several seconds. After setting the desired exposure time, an exposure program is selected. For this purpose, the MODE button on the keypad (3) is pressed, until the indicator lamps (10) marked as number 3 being equivalent of the desired 600 Hz pulsating light exposure program illuminate. After selecting the desired exposure parameters, the START button on the keypad (3) is pressed to begin the process of exposure of the body area being subject to the light therapy. The end of exposure is indicated by an aural signal and the exposure completes automatically after a selected exposure time ends. The ON/OFF button on the keypad (3) is used to turn off the device.

## Claims

1. The light-emitting diode (LED) light therapy device is characteristic by its feature of being equipped with a matrix board (1) and a control system board (2), connected to a keypad (3), while the matrix board (1) comprises light-emitting diodes (LEDs) in the red light wavelength range (LED-R) (4) and the infrared light wavelength range (LED-IR) (5). It is equipped with a power supply socket (6), a battery socket (7) and cables (8) connecting the matrix board (1) with the control system board (2) equipped with a pre-programmed processor (11), which controls the LED diodes within the red light wavelength range (LED-R) (4) and the infrared wavelength range (LED-IR) (5), a buzzer (9) and indicator lamps (10), and the LED diodes within the red light wavelength range (LED-R) (4) and the infrared wavelength range (LED-IR) (5), the buzzer (9) and the indicator lamps (10) are controlled via the keypad (3) equipped with the ON/OFF button used to switch on/off the device, the TIME button used to select the exposure time, the MODE button used to select the exposure program, the START button used to start the exposure process with the parameters already selected by the TIME and MODE buttons, and points through which light reaches the indicator lights (10).

2. The device according to claim No. 1 is characteristic by the LED diodes, the structure of which is based on GaAlAs semiconductors generating electromagnetic waves within the red light wavelength range and GaAlAs/GaAs semiconductors generating electromagnetic waves within the close infrared light wavelength range that are favourably used.

3. The device according to claim No. 1 is characteristic by the LEDs favourably of the wavelength of 625-680 nm and the radiation angle of 10-30 degrees that are used as the red light wavelength range LEDs (LED-R) (4).

4. The device according to claim No. 1 is characteristic by the LEDs favourably of the light wavelength of 880 nm and the radiation angle of 30-45 degrees that are used as the infrared light wavelength range LEDs (LED-IR) (5).

5. The device according to claim No. 1 is characteristic by the matrix board (1) favourably comprising 108 light-emitting diodes (LEDs), whereas the quantitative ratio of the red light wavelength range LEDs (LED-R) (4) to the infrared light wavelength range LEDs (LED-IR) (5) is 1:1.

6. The device according to claim No. 1 is characteristic by the red light wavelength range LED diodes (LED-R) (4) and the infrared light wavelength range LED diodes (LED-IR) (5) that are alternately arranged on the matrix board (1) in a chequered pattern.
